# EUROPEAN PATENT APPLICATION

(11) **EP 2 845 576 A1**
(43) Date of publication of application: **11.03.2015**
(21) Application number: 12868492.5
(22) Date of filing: 01.08.2012
(51) Int. Cl.: A61J 1/10, A61J 1/05

(54) **DOUBLE-HEADED TRANSFUSION CONTAINER**

(30) Priority: 17.02.2012 CN 201220052322 U; 28.02.2012 CN 201220068045 U
(71) Applicant: Chongqing Lummy Pharmaceutical Co., Ltd., Chongqing 401123 (CN)
(72) Inventor: QIU, Yu, Chongqing 401123 (CN); LI, Ke, Chongqing 401123 (CN)
(74) Representative: Wilson, Gary
(86) International application number: PCT/CN2012/079495
(87) International publication number: WO 2013/120341

(57) **Abstract**

A double-headed transfusion container comprises a body portion (1), both the top and the bottom of the body portion (1) being provided with an opening portion (2). The body portion (1) and the opening portion (2) are integrally formed of polypropylene blending plastic through blow molding, so as to obtain a seamless transparent polypropylene blending plastic transfusion container, which has advantages of a light weight and a low cost, further has good self-draining performance, and can steadily drain the liquid in the container from the container without any vent needle. Both the top and the bottom of the body portion (1) are provided with the opening portion (2), which facilitates connection to another independent auxiliary instrument better and can improve the safety of the transfusion product.

## Description

### TECHNICAL FIELD

The present utility model relates to a transfusion container, and particularly to a double-headed transfusion container formed integrally of polypropylene blending plastic as material through blow molding.

### BACKGROUND

At present, transfusion containers used in the market mainly include transfusion container types such as a glass transfusion bottle, a plastic transfusion bottle, a non-PVC multilayer co-extrusion film soft bag, etc. The glass transfusion bottle has disadvantages such as a brittle nature, a large weight, inconvenient for transportation and unfavorable for recovery, and has a less and less market share. The disadvantage of the plastic transfusion bottle is that it is difficult to completely drain the liquid within the container, and an intake needle is required to be used to ensure balance of pressures inside and outside the bottle. The non-PVC multilayer co-extrusion film soft bag requires no air needle when being used and can inwardly contracts and deforms freely under the effect of external atmospheric pressure, to self-drain the liquid within the container, which can prevent ambient air from contaminating medicine, thereby guaranteeing the efficacy of the medicine and health of a patient. However, the non-PVC multilayer co-extrusion film soft bag has the disadvantages that the hot fusion quality is not stable and leakage is susceptible to occur in the product, thereby causing scraps, a lowered qualified rate, and a higher production cost.

Furthermore, the above transfusion container is provided with an opening portion at the top of a body portion, and usually provided with an accessory such as a ring at the bottom. The opening portion at the top may be welded to a double valve cover for sealing and transfusing. The accessory at the bottom is used for suspending or fixing the transfusion container. With the increasing market demand and increasing consumer awareness for safety, more and more auxiliary transfusion products are available, such as more sanitary and safer doser, most of these auxiliary devices are disposable products, and are not suitable for being detached from the transfusion containers after use, since which may inevitably cause leakage or contamination. However, in the above traditional single port or unilateral multi-port transfusion containers, the auxiliary device can not be applied to some transfusion containers due to limitation of the number of interfaces (or bottle ports) of the transfusion containers, and some transfusion containers are not compatible with the corresponding auxiliary devices since being hindered by the spacing or position of the interface (or bottle port), such that there are bottlenecks in the connection between the traditional transfusion containers and other independent auxiliary devices (e.g., if being equipped with a medicine mixer having double needles).

### SUMMARY OF THE UTILITY MODEL

In view of the above disadvantages existing in the conventional technology, a double-headed transfusion container is provided according to the present utility model, which has a high yield, a high qualified rate, a lighter weight and is easy to connect to other independent auxiliary devices.

To address the above technical issues, the following technical solutions are employed according to the present utility model:

A double-headed transfusion container, wherein a top and a bottom of the body portion are provided with an opening portion, the body portion and the opening portion are integrally formed of polypropylene blending plastic through blow molding.

As a preferred solution of the present utility model, a first annular boss is formed on an outer circle of a port of the opening portion by projecting outwardly.

As another preferred solution of the present utility model, a second annular boss is formed on the outer circle of the opening portion and close to the body portion by projecting outwardly.

As yet another preferred solution of the present utility model, the outer diameter of the first annular boss is larger than the outer diameter of the second annular boss.

As an improved solution of the present utility model, the body portion has a flexible flat shape, the body portion has a first side wall and a second side wall, and a first face portion and a second face portion; a first convex portion and a second convex portion are located between the opening portion at the top and the body portion, and a third convex portion and a fourth convex portion are located between the opening portion at the bottom and the body portion; a first continuous contraction surface is formed between the first convex portion, the first side wall and the third convex portion, and a second continuous contraction surface is formed between the second convex portion, the second side wall and the fourth convex portion, the first contraction surface and the second contraction surface have a wall thickness smaller than other parts of the body portion.

As another improved solution of the present utility model, the first convex portion and the second convex portion are symmetrical relative to the body portion, and the third convex portion and the fourth convex portion are symmetrical relative to the body portion.

As yet another improved solution of the present application, an interface is provided on the opening portion at the top or/and the bottom of the body portion, a cross section of an inner bore of the interface is provided with a diaphragm.

As a further improved solution of the present application, a first annular welding platform is formed on an outer circle of one port of the interface by projecting outwardly.

As a further improved solution of the present application, a second annular welding platform is formed on an outer circle of another port of the interface by projecting outwardly.

The present utility model has the advantageous effects that the body portion and the opening portion of the double-headed transfusion container are integrally formed of polypropylene blending plastic through blow molding so as to successfully achieve blow molding a seamless transparent polypropylene blending plastic transfusion container, which has advantages of a good sealing, a high yield, a high qualified rate, a smaller weight and a low cost, and it can steadily drain the liquid in the container from the container without any vent needle and therefore having a good self-draining performance, and achieving a use effect of a soft bag, and it may transfuse hermetically without introducing outside air, and thus may effectively prevent particles and microbes in the air from causing secondary contamination to the liquid. Meanwhile, the top and bottom of the body portion are both provided with the opening portion, which is more conducive to connecting with other independent auxiliary devices (e.g., if being equipped with a medicine mixer having double needles), and meets the requirements of improving the safety of the transfusion products.

### DESCRIPTION OF THE DRAWINGS

Figure 1 is a front view of a double-headed transfusion container;
Figure 2 is a left view of the double-headed transfusion container; and
Figure 3 is a top view of the double-headed transfusion container.

In the drawings:

| | | | |
|---|---|---|---|
| 1 | body portion; | 2 | opening portion; |
| 3 | first annular boss, | 4 | second annular boss; |
| 21 | first side wall; | 22 | second side wall; |
| 23 | first face portion; | 24 | second face portion; |
| 25 | first convex portion; | 26 | second convex portion; |
| 27 | third convex portion; | 28 | fourth convex portion; |
| 29 | first contraction surface; | 30 | second contraction surface; |
| 5 | interface; | 6 | diaphragm; |
| 7 | first annular welding platform; and | 8 | second annular welding platform. |

### DETAILED EMBODIMENTS

The present utility model is further described in detail hereinafter in conjunction with drawings and embodiments.

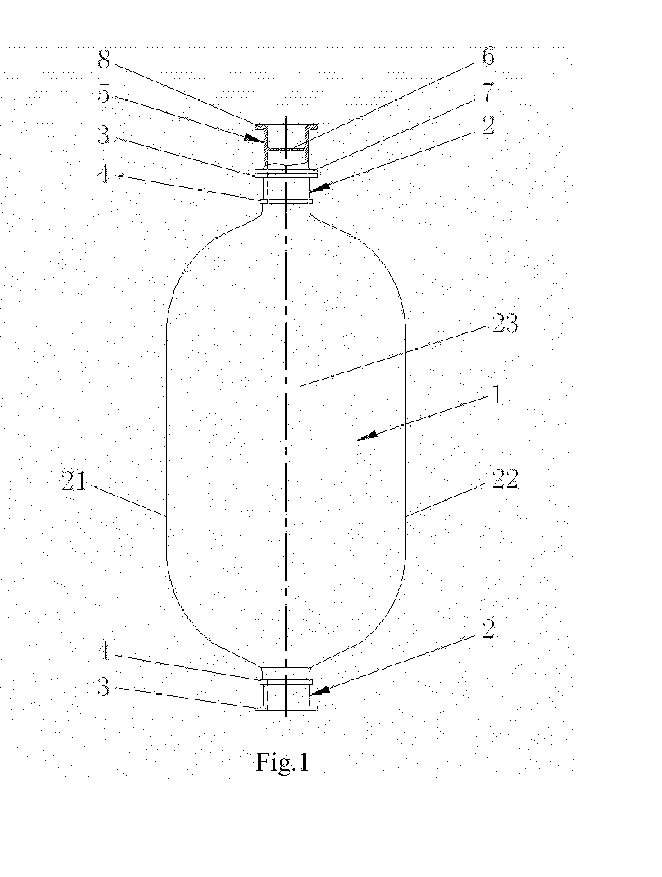

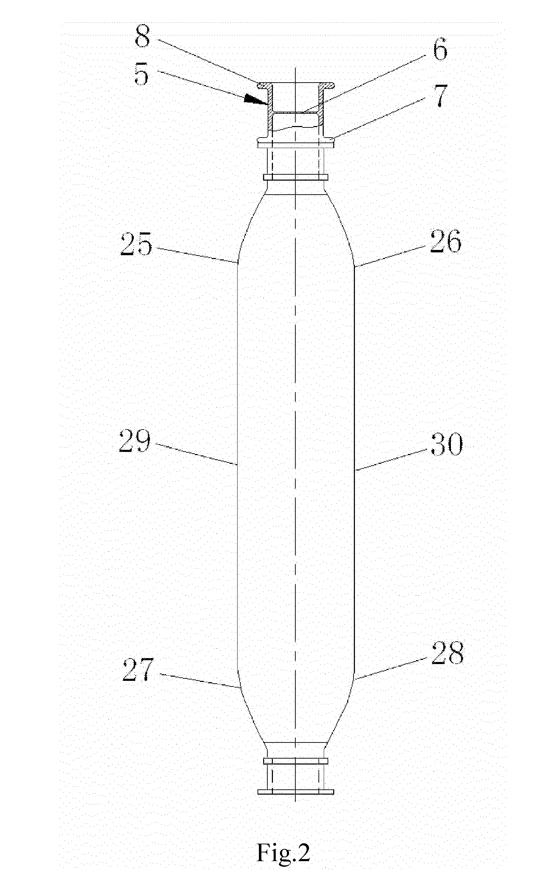

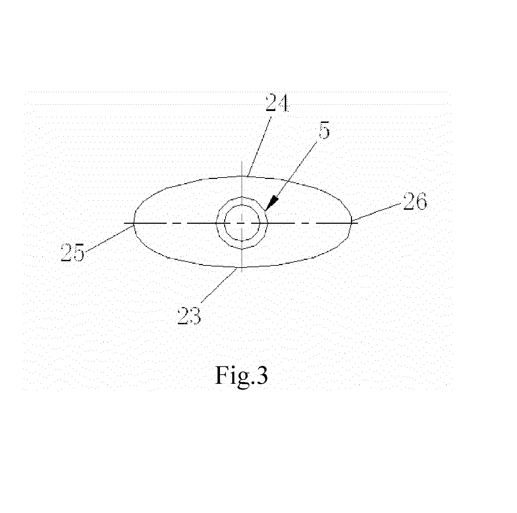

As shown in Figures 1, 2 and 3, a double-headed transfusion container includes a body portion 1, a top and a bottom of the body portion 1 are both provided with an opening portion 2, and the body portion 1 and the opening portion 2 are integrally formed of polypropylene blending plastic through blow molding. The body portion 1 and the opening portion 2 being integrally formed of polypropylene blending plastic through blow molding successfully achieves a blow molding seamless transparent polypropylene blending plastic transfusion container, which has advantages of a high yield, a high qualified rate, a smaller weight and a low cost.

A first annular boss 3 is formed on an outer circle of a port of the opening portion 2 by projecting outwardly, and the first annular boss 3 is mainly for connecting with other independent auxiliary devices (such as an interface 5, a double-valve-cover, a doser) by welding. A second annular boss 4 is formed on an outer circle of the opening portion 2 and close to the body portion 1 by projecting outwardly, for enhancing the strength of the opening portion 2. The outer diameter of the first annular boss 3 is larger than the outer diameter of the second annular boss 4.

The body portion 1 has a flexible flat shape, and it has a first side wall 21 and a second side wall 22, and a first face portion 23 and a second face portion 24. A first convex portion 25 and a second convex portion 26 are located between the opening portion 2 at the top and the body portion 1, and a third convex portion 27 and a fourth convex portion 28 are located between the opening portion 2 at the bottom and the body portion 1. A first continuous contraction surface 29 is formed between the first convex portion 25, the first side wall 21 and the third convex portion 27, and a second continuous contraction surface 30 is formed between the second convex portion 26, the second side wall 22 and the fourth convex portion 28, the first contraction surface 29 and the second contraction surface 30 have a wall thickness smaller than other parts of the body portion 1. The third convex portion 27 and the fourth convex portion 28 may substantially synchronously contract and deform inwardly along the first contraction surface 29 and the second contraction surface 30 with continuously draining of the liquid in the transfusion container, and the contractions and deformations respectively extend gradually toward the first convex portion 25 and the second convex portion 26. The first face portion 23 and the second face portion 24 may get close together from the end of the opening portion 2 at the bottom and have the abutment extend toward the direction of the opening portion 2 at the top with continuously draining of the liquid in the transfusion container.

The first convex portion 25 and the second convex portion 26 are symmetrical relative to the body portion 1, and the third convex portion 27 and the fourth convex portion 28 are symmetrical relative to the body portion 1, which achieves that the transfusion container uniformly contracts and deforms along the first contraction surface 29 and the second contraction surface 30 effectively, thereby achieving that the first face portion 23 and the second face portion 24 of the body portion 1 abut to each other at as large area as possible.

An interface 5 is provided on the opening portion 2 at the top or/and the bottom of the body portion 1, and in the present example, the interface 5 is provided only on the opening portion 2 at the top of the body portion 1, a cross section of an inner bore of the interface 5 is provided with a diaphragm 6. A first annular welding platform 7 is formed on an outer circle of one port of the interface 5 by projecting outwardly, and a second annular welding platform 8 is formed on an outer circle of another port of the interface 5 by projecting outwardly. The first annular welding platform 7 is welded to the first annular boss 3 located on the opening portion 2 of the transfusion container, and the second annular welding platform 8 is welded to the other accessories (such as a double-valve-cover or a doser). The first annular welding platform 7 and the second annular welding platform 8 may increase the welding area and thus increasing the strength of the welding with the opening portion of the transfusion container and other accessories. Meanwhile, the diaphragm 6 within the interface 5 enables a passage below the diaphragm 6 and the interior of the transfusion container to have a high level of cleanliness before use. When an upper port of the interface 5 is used alone, just using a dosing needle and a transfusing needle to pierce the diaphragm 6, mixing medicine and/or transfusion may be realized. A piercing position of the dosing needle may also be limited by an inner wall of the interface 5, using it alone may simplify processes and reduce costs. Of course, the upper port of the interface 5 may also be used in a state of being welded with other accessories (just welding a medicine mixer or the like to the upper port of the interface 5), thus mixing medicine and/or transfusion may be achieved.

When the double-headed transfusion container is used, the interface 5 at the top of the body portion 1 may be used as a medicine mixing end, and the opening portion 2 at the bottom of the body portion 1 may be used as a transfusion end, this way is more conducive to connecting with other independent auxiliary devices (e.g., if being equipped with a medicine mixer having double needles) and meets requirements for improving the safety of the transfusion products. In a case that the interface 5 at the top of the body portion 1 is connected with the medicine mixer, a medicine container is allowed to still remain in the medicine mixer after the medicine is mixed, which may effectively monitor the dispensing prescription and reduce the confusion error caused by dispensing negligence, thereby effectively ensuring the patient's medication safety. A transfusion hollow needle may pass through a rubber plug of a combining cover to be inserted into the liquid in the transfusion container, a discharge valve is adjusted to an appropriate position, such that the flow rate of the draining liquid is stabilized. Under the action of the external atmospheric pressure, the transfusion container begins to deform and contract inwardly along the first contraction surface 29 and a second contraction surface 30 and concave inwardly from the end of the body portion 1 close to the port 2 at the bottom of the body portion 1. With continuously draining of the liquid, the first face portion 23 and the second face portion 24 of the body portion 1 gradually abut together and the abutment gradually extends toward the end of the opening portion 2 at the top, the area of the abutment of the first face portion 23 and the second face portion 24 gets bigger and bigger, until all the liquid in the container is drained. Thereby, the liquid in the container can be steadily drained from the container without any vent needle to achieve self-draining.

Finally, it should be noted that, the above examples are only used for illustrating the technical solutions of the present application, and are not interpreted as limitation, although the present utility model is described in detail in conjunction with the preferred examples, it should be understood that, for those skilled in the art, modifications or equivalent substitutions may be made to the technical solutions of the present utility model, without departing from the spirit and scope in the technical solutions of the present utility model, which should be included within the scope of the present utility model defined by the claims.

## Claims

1. A double-headed transfusion container, comprising a body portion (1), **characterized in that** the top and the bottom of the body portion (1) are provided with an opening portion (2), the body portion (1) and the opening portion (2) are integrally formed.

2. The double-headed transfusion container according to claim 1, **characterized in that** a first annular boss (3) is formed on an outer circle of a port of the opening portion (2) by projecting outwardly.

3. The double-headed transfusion container according to claim 2, **characterized in that** a second annular boss (4) is formed on the outer circle of the opening portion (2) and close to the body portion (1) by projecting outwardly.

4. The double-headed transfusion container according to claim 3, **characterized in that** the outer diameter of the first annular boss (3) is larger than the outer diameter of the second annular boss (4).

5. The double-headed transfusion container according to the claim 3, **characterized in that** the body portion (1) has a flexible flat shape, the body portion (1) has a first side wall (21) and a second side wall (22), and a first face portion (23) and a second face portion (24); a first convex portion (25) and a second convex portion (26) are located between the opening portion (2) at the top and the body portion (1), and a third convex portion (27) and a fourth convex portion (28) are located between the opening portion (2) at the bottom and the body portion (1); a first continuous contraction surface (29) is formed between the first convex portion (25), the first side wall (21) and the third convex portion (27), and a second continuous contraction surface (30) is formed between the second convex portion (26), the second side wall (22) and the fourth convex portion (28), the first contraction surface (29) and the second contraction surface (30) have a wall thickness smaller than other parts of the body portion (1).

6. The double-headed transfusion container according to claim 5, **characterized in that** the first convex portion (25) and the second convex portion (26) are symmetrical relative to the body portion (1), and the third convex portion (27) and the fourth convex portion (28) are symmetrical relative to the body portion (1).

7. The double-headed transfusion container according to any one of claims 1 to 6, **characterized in that** an interface (5) is provided on the opening portion (2) at the top or/and the bottom of the body portion (1), a cross section of an inner bore of the interface (5) is provided with a diaphragm (6).

8. The double-headed transfusion container according to claim 7, **characterized in that** a first annular welding platform (7) is formed on an outer circle of one port of the interface (5) by projecting outwardly.

9. The double-headed transfusion container according to claim 7, **characterized in that** a second annular welding platform (8) is formed on an outer circle of another port of the interface (5) by projecting outwardly.
